# EUROPÄISCHE PATENTSCHRIFT

(11) **EP 2 396 052 B1**
(45) Veröffentlichungstag und Bekanntmachung des Hinweises auf die Patenterteilung: **15.07.2020**
(21) Anmeldenummer: 10711320.1
(22) Anmeldetag: 11.02.2010
(51) Int. Cl.: A61M 1/16, A61M 1/34, B01D 63/02

(54) **VORRICHTUNG ZUR BEHANDLUNG EINER BIOLOGISCHEN FLÜSSIGKEIT**
DEVICE FOR THE TREATMENT OF A BIOLOGICAL FLUID
DISPOSITIF DE TRAITEMENT D'UN LIQUIDE BIOLOGIQUE

(30) Priorität: 12.02.2009 DE 102009008601
(43) Veröffentlichungstag der Anmeldung: 21.12.2011
(73) Patentinhaber: Novalung GmbH, 74388 Talheim (DE)
(72) Erfinder: MAURER, Andreas, 72072 Tübingen (DE)
(74) Vertreter: Graf von Stosch, Andreas
(86) Internationale Anmeldenummer: PCT/EP2010/000852
(87) Internationale Veröffentlichungsnummer: WO 2010/091867

(56) Entgegenhaltungen:
- WO-A1-2006/057473
- DE-A1- 10 017 690
- US-A- 3 989 626
- US-A- 4 405 688

## Beschreibung

### Hintergrund der Erfindung

Die Erfindung betrifft eine Vorrichtung zur Behandlung einer biologischen Flüssigkeit und insbesondere eine Vorrichtung mit einer Kammer, die zur Aufnahme der biologischen Flüssigkeit bestimmt ist, und einer weiteren Kammer, die zur Aufnahme eines Gases bestimmt ist, wobei die Kammern durch eine gasdurchlässige Membran voneinander getrennt sind, und wobei die Membran einen Transfer von Gasmolekülen zwischen den Kammern ermöglicht.

Dabei handelt es sich um eine Begasungs- bzw. Entgasungsvorrichtung, bei der ein oder mehrere Gase von einem Medium in ein anderes übertreten können, oder eine Gasaustauschvorrichtung, die den Austausch eines oder mehrerer Gase zwischen zwei Medien ermöglicht. Solche Vorrichtungen finden Anwendung in der Chemie, der Biotechnologie und der Medizin. Ein wichtiger Einsatzzweck in der Medizin ist die Anreicherung des Blutes mit Sauerstoff und/oder die Entfernung von Kohlenstoffdioxid aus dem Blut. Solche Maßnahmen sind bspw. bei diversen Operationen und bei der Behandlung von verschiedenen Lungenerkrankungen notwendig.

Die derzeit einzige langfristig effektive Therapieoption für Patienten mit endgradiger funktioneller Lungenerkrankung stellt die Lungentransplantation dar. Eine andere medizinische Lösung, um dauerhaft die Funktion der Lunge zu ersetzen, existiert hingegen nicht. Bei Patienten, die unter chronischen Lungenerkrankungen leiden und nicht für eine Lungentransplantation in Betracht kommen, besteht ein Bedürfnis nach künstlichen Lungenersatzverfahren. Ein Beispiel hierfür sind Frühgeborene, die typischerweise eine Unterstützung ihrer Lungenfunktion über mehrere Wochen oder sogar über Monate brauchen. Außerdem besteht ein Bedürfnis nach Lungenersatzvorrichtungen bei Patienten, die auf eine Lungentransplantation warten.

Patienten, die mit künstlichen Lungenersatzverfahren behandelt werden müssen, leiden jedoch häufig auch unter einer zusätzlichen Niereninsuffizienz. Krankheiten, die zu Lungenversagen führen, können große Volumen von flüssiger Reanimation erfordern und die Patienten erhalten oft erhebliche Mengen von Blutprodukten. Die daraus entstehende körperliche Überladung mit Flüssigkeiten kann eventuell zu einer allgemeinen Verschlechterung des Patientenzustandes, zu einem Lungenödem oder sogar zu einem kompletten Organversagen führen. Solche Patienten werden deshalb an einem zusätzlichen Hämofiltrations- bzw. Hämodialysegerät angeschlossen. Dies führt jedoch zu einer verwickelten Konstellation der Behandlungsgeräte und einer größeren Belastung des Gefäßsystems der Patienten.

WO 2006/057473, DE 100 17 690 A1, US 4,405,688 A und US 3,989,626 A beschreiben Materialien und Vorrichtungen, die zur Sauerstoffanreicherung von Blut verwendet werden können. Keines dieser Dokumente offenbart jedoch eine Vorrichtung, die eine Mehrzahl von zweiten Kammern (mit einer gasdurchlässigen und flüssigkeitsundurchlässigen Membran) und eine Mehrzahl von dritten Kammern (mit einer flüssigkeitsdurchlässigen) Membran aufweist.

### Kurze Beschreibung der Erfindung

Der vorliegenden Erfindung liegt die Aufgabe zugrunde, eine verbesserte Vorrichtung zur Behandlung einer biologischen Flüssigkeit bereitzustellen, welche in der Chemie, der Biotechnologie oder der Medizin, insbesondere zur Begasung oder Entgasung bzw. zum Gasaustausch im Blut, Verwendung finden kann.

Diese Aufgabe wird durch eine Vorrichtung gemäß Anspruch 1 gelöst. Bevorzugte Merkmale der Erfindung sind in den abhängigen Ansprüchen angegeben.

Erfindungsgemäß wird eine Vorrichtung zur Behandlung einer biologischen Flüssigkeit bereitgestellt, umfassend mindestens drei Kammern, wobei eine erste Kammer, die zur Aufnahme der biologischen Flüssigkeit bestimmt ist, und eine zweite Kammer, die zur Aufnahme eines Gases bestimmt ist, durch mindestens eine gasdurchlässige und flüssigkeitsundurchlässige Membran voneinander getrennt sind, welche Membran einem Transfer von Gasmolekülen zwischen den ersten und zweiten Kammern dient, und wobei die erste Kammer und eine dritte Kammer durch mindestens eine flüssigkeitsdurchlässige Membran voneinander getrennt sind, die einem Transfer von einer oder mehreren Komponenten zwischen den ersten und dritten Kammern dient.

Die jeweiligen Membranen sind typischerweise halbdurchlässig oder semipermeabel und besitzen normalerweise Poren, deren Größe die Funktion und den Effekt der jeweiligen Membran bestimmen. Mit anderen Worten kann jede Membran einerseits eine poröse Membran sein, d.h. eine Membran, die diskrete Poren aufweist. Andererseits kann die Membran eine homogene Löslichkeitsmembran ohne diskrete Poren sein, in der der Stofftransport durch Lösung des Permeats (z.B. des Gases) im Polymer erfolgt und die Trennung aufgrund unterschiedlicher Löslichkeiten im Polymer stattfindet. Vorzugsweise ist die Membran eine nicht-poröse permeable Membran. Der Gasaustausch kann dem konvektiven und diffusiven Stoffaustausch unterliegen. Vorzugsweise ist der Gasaustausch diffusiv und wird über die Differenz der Gaskonzentration auf beiden Seiten der Membran bestimmt.

In einer bevorzugten Ausführungsform der Erfindung ist die erste Kammer als Durchflusskammer gestaltet und weist einen Einlass und einen Auslass auf. Beispielsweise ist mindestens eine der zweiten und dritten Kammern ebenso als Durchflusskammer gestaltet. Die erste Kammer ist bevorzugt zum Durchfluss in einer Richtung gegen oder quer zur Durchflussrichtung der zweiten bzw. der dritten Kammer bestimmt.

Die zweite Kammer ist von der mindestens einen gasdurchlässigen und flüssigkeitsundurchlässigen Membran abgegrenzt bzw. umschlossen. In ähnlicher Weise ist die dritte Kammer von der mindestens einen flüssigkeitsdurchlässigen Membran abgegrenzt bzw. umschlossen.

Die Membranen zwischen den jeweiligen Kammern bilden Trenn- oder Berührungsflächen, an denen der Transfer bzw. Übergang der Moleküle oder Komponenten von einer Kammer zur anderen stattfindet. An der mindestens einen gasdurchlässigen und flüssigkeitsundurchlässigen Membran zwischen den ersten und zweiten Kammern kann z. B. eine Begasung und/oder Entgasung der biologischen Flüssigkeit durchgeführt werden. Ferner kann an der mindestens einen flüssigkeitsdurchlässigen Membran, die die ersten und dritten Kammern voneinander trennt, eine zusätzliche Behandlung der biologischen Flüssigkeit durchgeführt werden. Da die erste, für die biologische Flüssigkeit bestimmte Kammer eine membranartige Trenn- bzw. Berührungsfläche sowohl mit der zweiten Kammer als auch mit der dritten Kammer aufweist, können zwei verschiedene Behandlungen der biologischen Flüssigkeit mit der erfindungsgemäßen Vorrichtung zeitgleich durchgeführt werden. Mit anderen Worten bietet die erfindungsgemäße Vorrichtung einen Aufbau, der mehrere gleichzeitige Behandlungen der biologischen Flüssigkeit ermöglicht. Erfindungsgemäß ist mindestens eine und bevorzugt jede Kammer von der jeweiligen Membran bzw. von der Membranstruktur mindestens teileweise gebildet. In einer bevorzugten Ausführungsform der Erfindung ist jede der zweiten und dritten Kammern mindestens teilweise, und bevorzugt völlig, von der jeweiligen Membran abgegrenzt bzw. umschlossen.

Gemäß der vorliegenden Erfindung befindet sich die zweite Kammer in bzw. innerhalb der ersten Kammer, d.h. die zweite Kammer ist von der ersten Kammer im Wesentlichen umgeben. In ähnlicher Weise befindet sich die dritte Kammer in bzw. innerhalb der ersten Kammer, sodass die dritte Kammer von der ersten Kammer ebenfalls im Wesentlichen umgeben ist. Durch die Anordnung der zweiten und dritten Kammern innerhalb und im Wesentlichen umgeben von der ersten Kammer kann die Trennfläche zwischen den jeweiligen Kammern maximiert werden. Damit kann die gesamte umschließende, von der Membran gebildete Oberfläche der jeweiligen Kammern dem Transfer bzw. Übergang von Molekülen oder Komponenten zwischen der in der ersten Kammer enthaltenen biologischen Flüssigkeit und den zweiten und dritten Kammern dienen.

Aufgrund der Tatsache, dass die zweiten und dritten Kammern innerhalb der ersten Kammer angeordnet sind, fließt die biologische Flüssigkeit außerhalb der zweiten und dritten Kammern und um sie herum. Dies hat zur Folge, dass die Dimensionen bzw. Abmessungen der ersten Kammer verhältnismäßig groß gestaltet werden können, sodass die biologische Flüssigkeit nicht über langen Strecken durch einen engen Spalt oder Kanal gepresst werden muss. Damit können hohe Druckabfälle in der ersten Kammer vermieden werden. Ferner, wenn die erste Kammer zum Durchfluss der biologischen Flüssigkeit in einer Richtung quer zur zweiten und dritten Kammer bestimmt ist, können die zweiten und dritten Kammern innerhalb der ersten Kammer als statische Mischer wirken, was zu einem verbesserten Austausch bzw. Übergang zwischen der biologischen Flüssigkeit und den zweiten und dritten Kammern führen kann.

In einer bevorzugten Ausführungsform der Erfindung ist die mindestens eine gasdurchlässige und flüssigkeitsundurchlässige Membran selektiv im Wesentlichen für bestimmte Gase, und insbesondere für Sauerstoff und/oder Kohlenstoffdioxid, durchlässig. Diese Ausführungsform ist für eine Begasung der biologischen Flüssigkeit (z.B. Blut) mit Sauerstoff (in einem sogenannten Oxygenator) und/oder eine Entgasung der biologischen Flüssigkeit (z.B. Blut) von Kohlenstoffdioxid (in einem sogenannten Ventilator) besonders geeignet. Mit anderen Worten ist diese Membran für einen Austausch von Sauerstoff aus der zweiten Kammer in das in der ersten Kammer enthaltene Blut besonders geeignet. Zugleich kann diese Membran eine Entfernung von Kohlenstoffdioxid aus dem Blut in die zweite Kammer unterstützen bzw. ermöglichen. Die mindestens eine gasdurchlässige und flüssigkeitsundurchlässige Membran mag zudem nicht oder nur geringfügig für Stickstoff durchlässig sein.

In einer Ausführungsform der Erfindung ist die dritte Kammer für einen Unterdruck bezüglich der ersten Kammer bestimmt, um einen Transfer von flüssigen Komponenten aus der biologischen Flüssigkeit in die dritte Kammer zu unterstützen bzw. zu befördern. Die mindestens eine flüssigkeitsdurchlässige Membran zwischen den ersten und dritten Kammern ist vorzugsweise für ein Filtrationsverfahren, insbesondere ein Hämofiltrationsverfahren, geeignet. Dabei wird die Membran ausgewählt, sodass bestimmte Komponenten oder Fraktionen der biologischen Flüssigkeit durch die Membran transferierbar sind, und andere nicht. Mit anderen Worten sollte die sich zwischen den ersten und dritten Kammern befindliche, flüssigkeitsdurchlässige Membran nach der gewünschten Behandlung der biologischen Flüssigkeit bestimmt und ausgewählt werden.

In einem Hämofiltrationsverfahren wird z.B. Serum (d.h. hauptsächlich Wasser) vom Blut über die Membran abgepresst und größere Moleküle, wie Eiweiße und Blutzellen, werden zurückgehalten. Die dritte Kammer könnte an eine Pumpe oder einen Absaugapparat angeschlossen werden, um einen Unterdruck zu erzeugen. Alternativ oder zusätzlich könnte die biologische Flüssigkeit in der ersten Kammer unter einem (z.B. leichten) Überdruck stehen, um die gewünschte Beförderung der Komponente (z.B. Wasser) aus der biologischen Flüssigkeit in die dritte Kammer zu befördern.

Vorzugsweise ist die dritte Kammer zur Aufnahme einer Flüssigkeit bestimmt, um einen Transfer der Komponente(n) aus der biologischen Flüssigkeit in die dritte Kammer zu unterstützen bzw. zu befördern. Die mindestens eine sich zwischen den ersten und dritten Kammern befindliche, flüssigkeitsdurchlässige Membran ist vorzugsweise für ein Dialyseverfahren, insbesondere ein Hämodialyseverfahren, geeignet. Die dritte Kammer ist dabei vorzugsweise zur Aufnahme einer Dialyselösung bestimmt. Hier wird nach dem Prinzip des Konzentrationsausgleichs kleinmolekularer Substanzen zweier Flüssigkeiten verfahren, die durch die flüssigkeitsdurchlässige, semipermeable Membran getrennt sind (Osmose). Von der Membran getrennt befindet sich auf der einen Seite das Blut mit Nephro-toxinen, Elektrolyten wie Kalium und Phosphat sowie harnpflichtigen Substanzen. Auf der anderen Seite der Membran befindet sich eine keimfreie Lösung, deren Wasser durch Umkehrosmose aufbereitet wurde, die keine Abfallprodukte enthält und einen an den jeweiligen Bedürfnissen des Patienten orientierten Anteil an Elektrolyten aufweist. Die semipermeable Membran zwischen Blut und Dialyselösung besitzt Poren, die kleine Moleküle wie Wasser, Elektrolyte und harnpflichtige Substanzen (z. B. Harnstoff, Harnsäure) durchlassen, aber große Moleküle wie Eiweiße und Blutzellen zurückhalten. Es liegt dabei im Ermessen des Fachmanns, ein Membranmaterial mit der geeigneten spezifischen Permeabilität für ein bestimmtes Gas oder für bestimmte Moleküle bei der angestrebten Verwendung der erfindungsgemäßen Vorrichtung auszuwählen. Permeabilitätswerte für viele Membranmaterialien sind aus dem Stand der Technik bekannt. Die in der Vorrichtung verwendete gasdurchlässige und flüssigkeitsundurchlässige Membran kann z.B. aus beliebigen Materialien bestehen, die eine gute Gaspermeabilität (Gasdurchlässigkeit) aufweisen. Gute Gaspermeabilitäten ist beispielsweise gegeben bei Werten von über 100 ml/m², bevorzugt über 1.000, stärker bevorzugt über 5.000, noch stärker bevorzugt über 10.000 und am stärksten bevorzugt über 20.000 ml/m² pro Stunde bei 24 atm (d.h. über 20.000 ml/m² innerhalb von 24h je bar Druckdifferenz), und bei 25°C, 90% relativer Feuchte (RH) und einer Materialstärke von ca. 50 µm, je nach gewünschtem Zweck in Hinblick auf das jeweils gewünschte Gas (insbesondere bspw. Sauerstoff oder Kohlendioxid). Ferner ist die Membran im Wesentlichen flüssigkeitsundurchlässig, d.h. sie besitzt eine Feuchtigkeitspermeabilität von < 1.000, bevorzugt < 500, stärker bevorzugt < 100 und noch stärker bevorzugt < 10 g/m² (i.e. gH₂O/m²) in 24 h, 40°C, 90% RH.

Die Membran kann aus einem organischen oder einem anorganischen Material bestehen oder ein solches umfassen. Zu anorganischen Membranmaterialien zählen Glas, Keramik (z.B. Aluminiumoxid, Titandioxid oder Zirkoniumoxid), Metall, Silikon oder Kohlenstoff. Zu organischen Membranmaterialien gehören insbesondere Polymermaterialien wie etwa Polyacrylamide, Polyacrylnitrile, Polyamide, Polybenzimidazole, Polybutadiene, Polycarbonate, Polydimethylsiloxane, Polyethersulfone, Polyetherimide, Polyolefine, Polyethylenterephtalate, Polymethylmethacrylat, Polymethylpenten, Polyphenylenoxid, Polystyrol, Polysulfone, Polyvinylalkohol, Polyvinylchlorid, Polyvinylidenfluorid, andere halogenierte Kohlenwasserstoffe und Zellulose und zyklische Olefincopolymere (COC). In einer bevorzugten Ausführungsform der Erfindung besteht mindestens eine der Membranen aus einem organischen Material oder umfasst ein solches, wobei das organische Material bevorzugt ein Polymer, Polymerkomposit (d.h. eine Mischung verschiedener Polymere oder Copolymere) oder Polymerschichtung (d.h. Polymerlaminate) ist bzw. umfasst.

Das Material der mindestens einen gasdurchlässigen und flüssigkeitsundurchlässigen Membran ist bevorzugt ein Polyolefin, weiter bevorzugt Polymethylpenten (PMP). Das Material der mindestens einen flüssigkeitsdurchlässigen Membran kann auch ein Polyolefin sein, ist aber bevorzugt Polyethersulfone (PES). Die Membranen haben eine Wandstärke bspw. im Bereich von 10 µm bis 200 µm, und bevorzugt im Bereich von 20 µm bis 100 µm. Vorzugsweise haben die Membranen ein geeignetes Trägermaterial zur Stabilisierung. In einem bevorzugten Beispiel wird mindestens eine der Membranen, und bevorzugt jede Membran, durch ein Trägermaterial stabilisiert. Vorzugsweise umfasst die Membran eine stabilisierende Trägerschicht, ausgewählt aus der Gruppe bestehend aus porösen Schäumen, Keramiken, Polymeren, ggf. einer Stützschicht aus TPX. Die Membranen können auch mit einer äußeren Haut oder Schicht im Bereich von 0,1 µm bis 1 µm versehen werden, z.B. mit einer diffusiven Schicht.

Gemäß der Erfindung ist die zweite Kammer in mehreren Kammern unterteilt, bzw. umfasst die Vorrichtung mehrere zweite Kammern, die zur Aufnahme eines Gases bestimmt und von der ersten Kammer durch eine gasdurchlässige und flüssigkeitsundurchlässige Membran getrennt sind. Die mehreren zweiten Kammern befinden sich innerhalb der ersten Kammer bzw. sind von der ersten Kammer im Wesentlichen umgeben. Vorzugsweise haben die zweiten Kammern einen länglichen und bevorzugt im Wesentlichen zylinderförmigen Aufbau, der im Querschnitt einen oder mehrere durchgängige Hohlräume aufweist. Eine den Querschnitt abgrenzende Wand der zweiten Kammern bildet mindestens teilweise die gasdurchlässige und flüssigkeitsundurchlässige Membran.

In einer besonders bevorzugten Ausführungsform der Erfindung sind die mehreren zweiten Kammern in einer oder mehreren Reihen nebeneinander und bevorzugt auch in einem Abstand voneinander angeordnet. Ferner können die mehreren zweiten Kammern in mehreren Lagen angeordnet sein. Die zweiten Kammern sind beispielsweise als Hohlkörper, bevorzugt als Hohlfasern, ausgebildet, sodass die Wand jedes Hohlkörpers bzw. jeder Hohlfaser die gasdurchlässige und flüssigkeitsundurchlässige Membran bildet. Der Abstand zwischen den mehreren, nebeneinander angeordneten zweiten Kammern liegt bevorzugt im Bereich von 50 µm bis 1cm, weiter bevorzugt im Bereich von 100 µm bis 1 mm, und noch weiter bevorzugt im Bereich von 100 µm bis 500 µm. Dieser Abstand kann beliebig gewählt bzw. eingestellt werden.

In ähnlicher Weise ist die dritte Kammer in mehreren Kammern unterteilt, bzw. umfasst die Vorrichtung mehrere dritte Kammern, die durch eine flüssigkeitsdurchlässige Membran von der ersten Kammer getrennt und zum Entzug einer oder mehreren Komponenten der biologischen Flüssigkeit geeignet sind. Die mehreren dritten Kammern befinden sich innerhalb der ersten Kammer bzw. sind von der ersten Kammer im Wesentlichen umgeben. Vorzugsweise haben die dritten Kammern einen länglichen und bevorzugt im Wesentlichen zylinderförmigen Aufbau, der im Querschnitt einen oder mehrere durchgängige Hohlräume aufweist. Eine den Querschnitt abgrenzende Wand der dritten Kammern bildet mindestens teilweise die flüssigkeitsdurchlässige Membran.

In einer besonders bevorzugten Ausführungsform der Erfindung sind die mehreren dritten Kammern in einer oder mehreren Reihen nebeneinander und bevorzugt auch in einem Abstand voneinander angeordnet. Ferner können die mehreren dritten Kammern bevorzugt in mehreren Lagen angeordnet sind. Die dritten Kammern sind beispielsweise als Hohlkörper, bevorzugt als Hohlfasern, ausgebildet, sodass eine Wand jedes Hohlkörpers bzw. jeder Hohlfaser eine flüssigkeitsdurchlässige Membran bildet. Der Abstand zwischen den mehreren, nebeneinander angeordneten dritten Kammern liegt bevorzugt im Bereich von 50 µm bis 1cm, weiter bevorzugt im Bereich von 100 µm bis 1 mm, und noch weiter bevorzugt im Bereich von 100 µm bis 500 µm. Wie für die zweiten Kammer beschrieben, kann dieser Abstand beliebig gewählt bzw. eingestellt werden.

In einer bevorzugten Ausführungsform der Erfindung erstreckt sich eine längliche Ausrichtung der zweiten Kammer(n) quer, und bevorzugt rechtwinkelig, zu einer länglichen Ausrichtung der dritten Kammer(n). Wenn mehrere zweite Kammern in Reihen nebeneinander angeordnet sind und zusammen eine Lage bilden, und wenn mehrere dritte Kammern in Reihen nebeneinander angeordnet sind und zusammen auch eine Lage bilden, können diese Lagen derart auf einander gestapelt werden, dass eine längliche Ausrichtung der zweiten Kammern sich quer, und bevorzugt rechtwinkelig, zu einer länglichen Ausrichtung der dritten Kammern erstreckt.

Die kritischen Komponenten der Vorrichtung im Hinblick auf ihre Haltbarkeit sind die Membranen. Der bisherige vielseitige klinische Einsatz von organunterstützenden Systemen mit Fremdoberflächen, die mit Blut in Berührung kommen, hat gezeigt, dass es zu unerwünschten systemischen Reaktionen (z.B. eine proinflammatorische Immunantwort) kommen kann. Bei der Langzeitanwendung herkömmlicher Blutkontaktflächen führt eine Anlagerung von Plasmaproteinen und Zellen zur Querschnittsverengung und Thrombosebildung. Zudem wird bei langfristigem Einsatz die Bildung einer proliferativen Innenschicht bewirkt, was als "Neo-Intima" bezeichnet wird. Insbesondere wird dieses Phänomen bei Oxygenatoren, die zur Unterstützung der Lungenfunktion in beispielsweise Herz-Lungen-Maschinen Verwendung finden, aber auch bei Kunstherzsystemen bzw. Herzunterstützungssystemen oder Hämodialyseapparaturen beobachtet. Deshalb kann und sollte die Membran auf unterschiedliche Weise in ihrer Haltbarkeit verbessert werden. In einem Beispiel, kann die Membranen durch Plasmaaktivierung nachbehandelt werden.

In einem Beispiel, insbesondere für die Verwendung der Vorrichtung zur Behandlung einer biologischen Flüssigkeit in der Medizin, kann mindestens eine der Membranen, bevorzugt die mindestens eine gasdurchlässige und flüssigkeitsundurchlässige Membran, und weiter bevorzugt jede Membran, mit Zellen, vorzugsweise Epithelzellen, besiedelt sein. Für eine langfristig einsetzbare Gastransfervorrichtung im medizinischen Rahmen (beispielsweise als Lungenunterstützungssystem) stellt die Besiedlung der Membran mit Zellen eine deutliche Verlängerung der Haltbarkeit dar, da dadurch eine unspezifische Anlagerung von Substanzen aus den verwendeten Medien an die Membran vermieden bzw. stark gehemmt wird und somit die Membran in ihrer Gaspermeabilität mit der Zeit nicht oder nur unwesentlich verschlechtert wird. In einer bevorzugten Ausführungsform der Erfindung ist mindestens eine der Membranen, bevorzugt die mindestens eine gasdurchlässige und flüssigkeitsundurchlässige Membran, und weiter bevorzugt jede Membran (d.h. auch die mindestens eine flüssigkeitsdurchlässige Membran) mit einer oder mehreren Substanzen, ausgewählt aus der Gruppe, bestehend aus (Poly)-Saccharid, vorzugsweise Heparin, Nukleinsäure, Protein, vorzugsweise Albumin, beschichtet.

Zur Besiedlung der Membran mit Zellen bzw. zur Beschichtung der Membran mit anderen Substanzen kann es vorteilhaft oder auch notwendig sein, die Oberfläche der Membran vorher zu modifizieren. Verfahren zur Modifikation von Membranen sind aus dem Stand der Technik bekannt und können vom Fachmann anwendungsbezogen ausgewählt werden. Beispielsweise kann es notwendig sein, die Hydrophobizität / Hydrophilie / Ladungsdichte der Membran zu verändern, beispielsweise durch physikalische oder chemische Behandlung der Membran, um die Anhaftung zu verbessern.

In einer bevorzugten Ausführungsform der Erfindung ist die mindestens eine gasdurchlässige und flüssigkeitsundurchlässige Membran, die die ersten und zweiten Kammern voneinander trennt, typischerweise auf mindestens einer Seite strukturiert. Vorzugsweise ist die der biologischen Flüssigkeit in der ersten Kammer zugewandte Membranseite strukturiert. In diesem Zusammenhang wird auf die parallele internationale Patentanmeldung PCT/EP2009/006403 hingewiesen. Insbesondere bei Flüssigkeiten werden die positiven Eigenschaften (z.B. die Herabsetzung des Flusswiderstandes) einer strukturierten Membran auf den Gasübertritt deutlich. Die Geometrie der Strukturen auf der Membran kann beliebig variiert werden. So können Kanäle und/oder Verästelungen als Strukturen auf der Membran verwendet werden, die beispielsweise die Kapillarstruktur der natürlichen Lunge nachahmen.

### Kurze Beschreibung der Zeichnungen

Die Erfindung wird nun anhand bestimmter in den beigefügten Zeichnungen dargestellter Ausführungsbeispiele erläutert, in denen gleiche Merkmale mit den gleichen Bezugszeichen versehen sind. Es zeigt:
- Figur 1: eine schematische Seitenansicht einer Vorrichtung gemäß einem einfachen Ausführungsbeispiel der Erfindung,
- Figur 2: eine schematische Querschnitt der Vorrichtung gemäß dem einfachen Ausführungsbeispiel der Erfindung in Figur 1 in Richtung X-X,
- Figur 3: eine schematische isometrische Ansicht einer Vorrichtung gemäß einem bevorzugten Ausführungsbeispiel der Erfindung,
- Figur 4: eine DAPI-Färbung einer besiedelten Hohlfasern,
- Figur 5: die arteriellen CO₂-Werte von vier Versuchstieren,
- Figur 6: die arteriellen O₂-Werte der vier Versuchstiere,
- Figur 7: die venösen CO₂-Werte der vier Versuchstiere,
- Figur 8: die venösen O₂-Werte der vier Versuchstiere,
- Figur 9: die Delta 02- und CO₂-Werte für die Gasaustauschleistung der Prototypen nach dem Tierversuch,
- Figur 10: den mittleren Druckabfall über einen Test-Modul,
- Figur 11: die Haemofiltratvolumen für Tier 1 und Tier 3 aus den unbesiedelten Test-Modulen, und
- Figur 12: einen Vergleich der Serum- und Haemofiltratparameter.

### Beschreibung der Ausführungsbeispiele der Erfindung

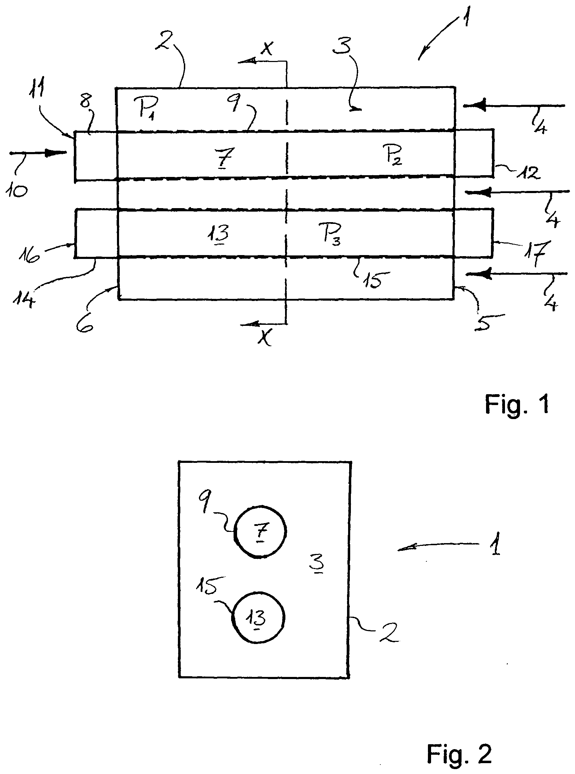

In den Figuren 1 und 2 ist ein einfaches Beispiel des erfinderischen Konzeptes dargestellt. Eine Vorrichtung 1 gemäß der Erfindung weist ein Gehäuse 2 auf, das eine erste Kammer 3 umschließt bzw. teilweise definiert. Die erste Kammer 3 ist zur Aufnahme einer biologischen Flüssigkeit (wie z.B. Blut) bestimmt und ist als eine Durchflusskammer ausgebildet. Die biologische Flüssigkeit bzw. das Blut fließt in die von den Pfeilen 4 gezeigte Richtung durch einen Einlass 5 im Gehäuse 2 in die erste Kammer 3 ein und verlässt die erste Kammer 3 durch einen gegenüberliegenden Auslass 6. Das Gehäuse 2 besteht vorzugsweise aus einem für die biologische Flüssigkeit chemisch nicht reagierenden Kunststoff, wie z.B. Polyethylen oder Polyurethan.

Ferner weist die Vorrichtung 1 eine rohrförmige zweite Kammer 7 auf, die sich durch die erste Kammer 3 erstreckt und von der ersten Kammer 3 im Wesentlichen umgeben ist. Eine rohrförmige Wand 8, die den Hohlraum der zweiten Kammer 7 umschließt, ist relativ dünn und besteht bevorzugt aus einem Kunststoff. Die Wand 8 dient als Trägermaterial für eine äußere Schicht, welche zusammen mit Wand 8 eine gasdurchlässige und flüssigkeitsundurchlässige Membran 9 bildet, sodass die rohrförmige Wand 8 einen Transfer von Gasmolekülen zwischen der ersten Kammer 3 und der zweiten Kammer 7 ermöglicht. Mit anderen Worten bildet die Membran 9 eine Trenn- bzw. Berührungsfläche, an der ein intimer Kontakt zwischen den molekularen Komponenten des Blutes und des in der zweiten Kammer enthaltenen Mediums zustande kommen bzw. stattfinden kann.

Die zweite Kammer 7 ist zur Aufnahme eines Gases wie z.B. Sauerstoff bestimmt und ist ebenso als Durchflusskammer ausgebildet. Die gasdurchlässige und flüssigkeitsundurchlässige Membran 9 ist bevorzugt selektiv für Sauerstoff und Kohlenstoffdioxid durchlässig. Der Sauerstoff fließt in die vom Pfeil 10 gezeigte Richtung durch einen Einlass 11 in die zweite Kammer 7 ein und verlässt die zweite Kammer 7 durch einen gegenüberliegenden Auslass 12. Der Sauerstoff, der durch die zweite Kammer 7 fließt, geht jedoch auch zum Teil durch die gasdurchlässige und flüssigkeitsundurchlässige Membran 9 in das durch die erste Kammer 3 fließende Blut über. Hierdurch findet eine Anreicherung des Blutes mit Sauerstoff statt. Auf ähnlicher Art und Weise kann ein Transfer bzw. eine Entfernung von Kohlenstoffdioxid aus dem Blut durch die Membran 9 in die zweite Kammer 7 stattfinden, sodass ein sogenanntes Ventilation- bzw. Lungenersatzverfahren an der Membran 9 erfolgt.

Der Druck P₁ und/oder die Strömung der biologischen Flüssigkeit bzw. des Blutes in der ersten Kammer 3 in Verhältnis zum Druck P₂ und/oder zur Strömung des durch die zweite Kammer 7 fließenden Sauerstoffes kann derart gewählt bzw. eingestellt werden, dass ein gewünschter Transfer von Sauerstoff ins Blut und/oder Kohlenstoffdioxid aus dem Blut erreicht wird. Das Blut kann z.B. mit einer Pumpe (nicht gezeigt) durch die erste Kammer 1 befördert werden oder es kann auch lediglich unter dem Druck des Kreislaufsystems des Patienten durch die erste Kammer 1 fließen.

Mit weiterem Bezug auf die Figuren 1 und 2 weist die Vorrichtung 1 ferner eine rohrförmige dritte Kammer 13 auf, die sich ebenso wie die zweite Kammer durch die erste Kammer 3 erstreckt und von der ersten Kammer 3 im Wesentlichen umgeben ist. Eine rohrförmige Wand 14, die den Hohlraum der dritten Kammer 13 umschließt, ist relativ dünn und besteht bevorzugt aus einem Kunststoff. Wie mit der zweiten Kammer 7, dient die Wand 14 als Trägermaterial für eine äußere Schicht, die zusammen mit der Wand 14 in diesem Fall jedoch eine flüssigkeitsdurchlässige Membran 15 bildet, sodass die rohrförmige Wand 14 einen Transfer von flüssigen Komponenten zwischen der ersten Kammer 3 und der dritten Kammer 13 ermöglicht. Insbesondere bildet diese Membran 15 eine Trenn- bzw. Berührungsfläche, welche einem Entzug einer oder mehreren flüssigen Komponenten der biologischen Flüssigkeit dient.

In einem bestimmten Ausführungsbeispiel ist die dritte Kammer 13 zum Anschluss an einen Absauggerät (nicht gezeigt) bestimmt, um einen leichten Unterdruck in der dritten Kammer 13 (bezüglich der ersten Kammer 3) zu erzeugen. Dieser Unterdruck unterstützt bzw. befördert einen Transfer von flüssigen Komponenten wie z.B. Wasser aus dem durch die erste Kammer 3 fließenden Blut in die dritte Kammer 13. Damit funktioniert die sich zwischen den ersten und dritten Kammern befindliche, flüssigkeitsdurchlässige Membran 15 als Filter, über den kleinere Moleküle wie Wasser vom Blut abgepresst und größere Moleküle wie Eiweiße und Blutzellen zurückgehalten werden.

In einer alternativen Ausführungsform ist die dritte Kammer 13 zur Aufnahme einer Dialyselösung bestimmt, um einen Transfer der Komponente(n) aus der biologischen Flüssigkeit in die dritte Kammer zu befördern. Als solche kann die dritte Kammer 13 auch als Durchflusskammer gestaltet werden, wobei die Dialyselösung unter Einfluss einer Pumpe (nicht gezeigt) von einem Einlass 16 durch die dritte Kammer 13 zu einem Auslass 17 in einem Gegenstrom zum Blut befördert wird. Wie oben schon erwähnt, wird hier nach dem Prinzip des Konzentrationsausgleichs kleinmolekularer Substanzen zweier Flüssigkeiten verfahren, die durch die flüssigkeitsdurchlässige, semipermeable Membran getrennt sind (Osmose). Die semipermeable Membran 15 zwischen Blut und Dialyselösung besitzt Poren, die kleine Moleküle wie Wasser, Elektrolyte und harnpflichtige Substanzen (z. B. Harnstoff, Harnsäure) durchlassen, aber große Moleküle wie Eiweiße und Blutzellen zurückhalten.

Die Vorrichtung 1 hat einen Aufbau, der mehrere gleichzeitige Behandlungen der biologischen Flüssigkeit (in diesem Fall Blut) ermöglicht. Erstens kann das Blut mit einer Anreicherung von Sauerstoff bzw. einer Entfernung von Kohlendioxid behandelt werden. Zweitens kann das Blut gleichzeitig mit einem Filtrations- bzw. Dialyseverfahren behandelt werden. Die Erfindung schafft damit eine Vorrichtung, mit der eine einfachere und effizientere Behandlung eines unter einer Lungen- und Niereninsuffizienz leidenden Patienten möglich wird. Ferner, dadurch dass die zweiten und dritten Kammern innerhalb und im Wesentlichen umgeben von der ersten Kammer angeordnet sind, hat die Vorrichtung 1 einen Aufbau, der die Trenn- bzw. Berührungsfläche der biologischen Flüssigkeit mit den jeweiligen Kammern maximiert. Die gesamte umschließende, von der Membran gebildete Oberfläche der jeweiligen zweiten und dritten Kammern dient somit dem Transfer bzw. dem Übergang von Molekülen oder Komponenten zwischen der in der ersten Kammer enthaltenen biologischen Flüssigkeit und den in den zweiten und dritten Kammern enthaltenen Medien.

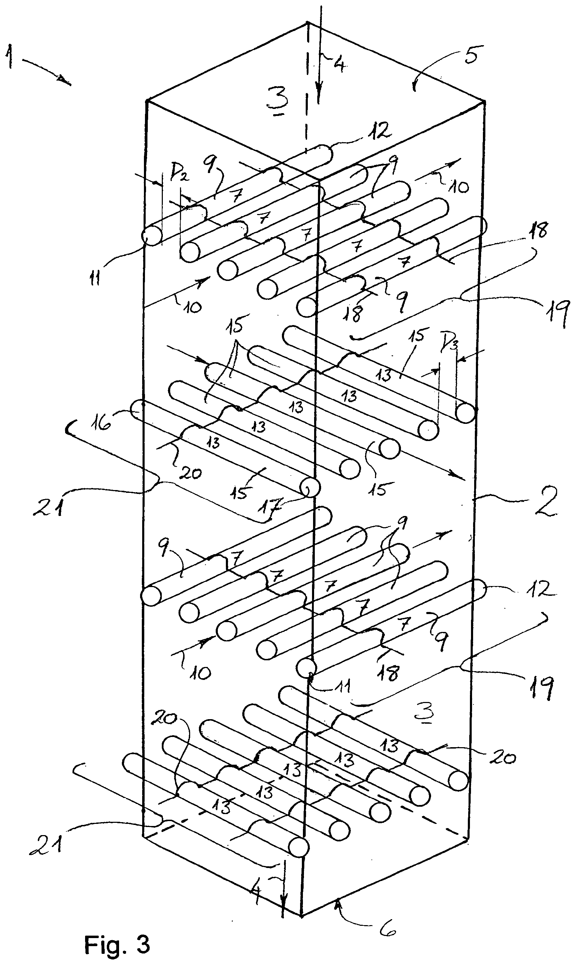

Mit Bezug auf die Figur 3 wird ein besonders bevorzugtes Ausführungsbeispiel der Erfindung nun erläutert. Die schematische Darstellung in Figur 3 zeigt eine erfindungsgemäße Vorrichtung 1 mit einem Gehäuse 2. Wie in dem ersten Ausführungsbeispiel umschließt bzw. definiert (zumindest teilweise) das Gehäuse 2 eine erste Kammer 3. Die erste Kammer 3 ist zur Aufnahme einer biologischen Flüssigkeit (wie z.B. Blut) bestimmt und ist als Durchflusskammer ausgebildet. Die biologische Flüssigkeit bzw. das Blut fließt in die vom Pfeil 4 gezeigte Richtung durch einen Einlass 5 im Gehäuse 2 in die erste Kammer 3 ein und verlässt die erste Kammer 3 durch einen gegenüberliegenden Auslass 6. Wie vorher, besteht das Gehäuse 2 vorzugsweise aus einem Kunststoff wie z.B. Polyethylen oder Polyurethan.

Die Vorrichtung 1 in Figur 3 weist mehrere rohrförmige zweite Kammern 7 auf, die nebeneinander in Reihen angeordnet sind. Die zweiten Kammern 7 erstrecken sich parallel durch die erste Kammer 3 und sind von der ersten Kammer 3 im Wesentlichen umgeben. In diesem Ausführungsbeispiel sind die rohrförmigen Wände 8, die die Hohlräume der zweiten Kammern 7 umschließen, in Form von Hohlfasern aus Polymethylpenten (PMP, auch als TPX bekannt), z.B. geschäumtes TPX. Diese Wände 8 und ihre äußeren Flächen bzw. Schichten bilden gasdurchlässige und flüssigkeitsundurchlässige Membranen 9, sodass einen Transfer von Gasmolekülen zwischen der ersten Kammer 3 und den sich im inneren der Hohlfasern befindlichen zweiten Kammern 7 ermöglicht. Die Membranen 9 bilden wieder Trenn- bzw. Berührungsflächen, an denen ein intimer Kontakt zwischen den molekularen Komponenten des Blutes und des in den zweiten Kammern enthaltenen Mediums zustande kommen bzw. stattfinden kann.

Die zweiten Kammern 7 sind zur Aufnahme eines Gases wie z.B. Sauerstoff bestimmt und sind ebenfalls als Durchflusskammern ausgebildet. Die gasdurchlässigen und flüssigkeitsundurchlässigen Membranen 9 sind selektiv für Sauerstoff und Kohlenstoffdioxid durchlässig. Der Sauerstoff fließt in die vom Pfeil 10 gezeigte Richtung durch Einlässe 11 in die zweiten Kammern 7 ein und verlässt die zweiten Kammern 7 durch gegenüberliegende Auslässe 12. Wie vorher, geht ein Teil des Sauerstoffes, der durch die zweiten Kammern 7 fließt, durch die gasdurchlässigen und flüssigkeitsundurchlässigen Membranen 9 in das durch die erste Kammer 3 fließende Blut über und führt zu einer Anreicherung des Blutes mit Sauerstoff. Auf ähnlicher Art und Weise findet ein Transfer bzw. eine Entfernung von Kohlenstoffdioxid aus dem Blut durch die Membranen 9 in die zweiten Kammern 7 statt, sodass ein sogenanntes Ventilation- bzw. Lungenersatzverfahren erfolgt.

Die in einer Reihe/Ebene nebeneinander angeordneten, die zweiten Kammern 7 bildenden TPX-Fasern werden in einem textiltechnischen Verfahren mit Kettfäden 18 miteinander verbunden. Daraus entsteht eine Art Fasermatte 19 mit definierten Abständen D₂ zwischen den Fasern. Dieser Abstand D₂ zwischen den Fasern dient dazu, dass das durch die erste Kammer 3 fließende Blut durch die Matte 19 durchfließen und damit einen maximalen Kontakt mit der Berührungsflächen der Membranen 9 erreichen kann. In diesem Ausführungsbeispiel haben die einzelnen TPX-Hohlfasern einen äußeren Durchmesser im Bereich von 100 µm bis 1 mm, bevorzugt im Bereich von 200 µm bis 600 µm, (z.B. einen Außendurchmesser von etwa 400 µm mit einer Wandstärke von etwa 100 µm), und sie sind in jeder Reihe bzw. Ebene nebeneinander mit einem Abstand D₂ im Bereich von 100 µm bis 500 µm angeordnet. Dieser Abstand kann beliebig gewählt werden. Daher wird klar, dass eine Vielzahl von TPX-Fasern nebeneinander gelegt und zu Matten 19 verarbeitet werden können. Die Abmessungen der TPX-Fasern-Membranmatte 19 betragen bspw. etwa 10 cm x 15 cm.

Nachdem die TPX-Hohlfasern zu Matten 19 verarbeitet werden, können diese Matten 19 anschließend weiterverarbeitet werden, in dem sie übereinander gestapelt werden. In Figur 3 werden lediglich zwei Lagen oder Matten 19 der zweiten, parallel verlaufenden Kammern 7 gezeigt und diese sind übereinander gestapelt. Es wird jedoch vom Fachmann verstanden, dass eine Vielzahl von solchen Matten 19 übereinander in der ersten Kammer 3 vorgesehen werden können. Obwohl in Figur 3 nicht so gezeigt, sind die Enden der die zweiten Kammern 7 bildenden TPX-Fasern zusammengebündelt bzw. zusammengeschaltet, sodass die einzelnen Einlässe 11 über eine gemeinsame Zufuhr mit Gas bzw. Sauerstoff gespeist werden können, und sodass die einzelnen Auslässe 12 in einen gemeinsamen Abfluss übergehen. Vorzugsweise gilt dies nicht nur für die zweiten Kammern 7 der einzelnen Matten 19 sondern für alle zweite Kammern 7 in allen Matten 19. Diese sogenannte "Zusammenschaltung" von den Fasern der gleichen Orientierung erfolgt vorzugsweise über ein Vergussverfahren, z.B. mit Polyurethan. Hier wird im äußeren Bereich des Faserstapels die Enden der Fasern mit flüssigem Kunststoff Umgossen. Nach Aushärtung des Kunststoffs wird dann von außen her scheibchenweise abgeschnitten, bis der Innenraum der Fasern eröffnet ist. Somit erreicht man eine gemeinsame Zufuhr in die einzelne Fasern bzw. Kammern.

Zudem weist die Vorrichtung 1 in Figur 3 mehrere rohrförmige dritte Kammern 13 auf, die sich ebenso wie die zweiten Kammern 7 parallel durch die erste Kammer 3 erstrecken und von der ersten Kammer 3 im Wesentlichen umgeben sind. In diesem Ausführungsbeispiel sind die rohrförmigen Wände 14, die die Hohlräume der dritten Kammern 13 umschließen, in Form von Hohlfasern aus Polyethersulfone (PES). Die Wände 14 der Hohlfasern mit deren äußeren Flächen bilden flüssigkeitsdurchlässige Membranen 15, welche einen Transfer von flüssigen Komponenten zwischen der ersten Kammer 3 und den sich im inneren der Hohlfasern befindlichen, dritten Kammern 13 ermöglichen. Insbesondere bilden die Membranen 15 Trenn- bzw. Berührungsflächen, welche einem Entzug einer oder mehreren Komponenten der biologischen Flüssigkeit dient.

Wie für die TPX-Fasern, werden die in einer Reihe bzw. Ebene nebeneinander angeordneten, die dritten Kammern 13 bildenden PES-Fasern in einem textiltechnischen Verfahren mit Kettfäden 20 miteinander verbunden. Daraus entsteht eine Art Fasermatte 21 mit definierten Abständen D₃ zwischen den PES-Fasern. Dieser Abstand D₃ zwischen den PES-Fasern dient auch dazu, dass das durch die erste Kammer 3 fließende Blut durch die Matte 21 durchfließen und damit einen maximalen Kontakt mit der Berührungsflächen der Membranen 15 erreichen kann. In Figur 3 werden lediglich zwei Lagen bzw. Matten 21 von dritten Kammern 13 gezeigt und diese sind als übereinander gestapelte Lagen 21 dargestellt. Wie mit den zweiten Kammern 7 haben die einzelnen PES-Fasern in diesem Ausführungsbeispiel einen äußeren Durchmesser im Bereich von 100 µm bis 1 mm, bevorzugt im Bereich von 200 µm bis 600 µm, (z.B. einen Außendurchmesser von etwa 500 µm mit einer Wandstärke von etwa 100 µm), und sie sind in jeder Reihe bzw. Ebene nebeneinander mit einem Abstand D₃ im Bereich von 100 µm bis 500 µm angeordnet. Eine Vielzahl von PES-Fasern können daher nebeneinander gelegt und zu Matten 21 verarbeitet werden. Die Abmessungen jeder PES-Faser-Membranmatte 21 betragen auch etwa 10 cm x 15 cm. Vorzugsweise haben die flüssigkeitsdurchlässigen Membranen eine maximale Porengröße ≤ 1 µm, und weiter bevorzugt ≤ 0,5 µm (z.B. maximale Porengröße ist 0,5 µm). Vorzugsweise wird mit diesen Membranen einen Transmembran-Flow von > 35 ml/[min.cm².bar] erzeugt.

Nachdem die PES-Hohlfasern zu Matten 21 verarbeitet sind, können die Matten 21 anschließend weiterverarbeitet werden, in dem sie übereinander gestapelt werden. In Figur 3 werden lediglich zwei Lagen oder Matten 21 der dritten, parallel verlaufenden Kammern 13 dargestellt. Es wird jedoch vom Fachmann verstanden werden, dass eine Vielzahl von solchen Matten 21 übereinander in der ersten Kammer 3 vorgesehen werden können.

Die Enden der die dritten Kammern 13 bildenden PES-Fasern werden zusammengebündelt bzw. zusammengeschaltet, sodass die einzelnen Einlässe 16 mit einem Unterdruck gemeinsam beaufschlagt oder über eine einzige Zufuhr mit einer Dialyselösung gemeinsam gespeist werden können, und sodass die einzelnen Auslässe 17 in einen gemeinsamen Abfluss übergehen. Diese sogenannte "Zusammenschaltung" von den Fasern der gleichen Orientierung erfolgt vorzugsweise über ein Vergussverfahren mit z.B. Polyurethan, wie in Zusammenhang mit den zweiten Kammern 7 beschrieben wurde. Dies gilt nicht nur für die dritten Kammern 13 der einzelnen Matten 21 sondern für alle dritten Kammern 13 in den gestapelten Matten 21.

Die längliche Ausrichtung der parallel angeordneten zweiten Kammern 7 verläuft rechtwinkelig zu der länglichen Ausrichtung der parallel angeordneten dritten Kammern 13 und die Lagen bzw. Matten 19, 21 der TPX- und PES-Fasern sind in diesem Beispiel abwechselnd unmittelbar auf- bzw. übereinander in einer relativ kompakten Anordnung gestapelt. Die Matten 19, 21 werden bevorzugt direkt aufeinander gelegt, sodass sie in Kontakt miteinander sind. In Figur 3 sind die Lagen bzw. Matten 19, 21 weit auseinander in einer "explodierten" Darstellung gezeigt, um der deutlichen Erklärung der Erfindung zu dienen. Die quadratische Anordnung der Hohlfasern und Lagen bzw. Matten 19, 21 in der Vorrichtung 1 ist für die Anschlüsse an den jeweiligen zweiten und dritten Kammern 7, 13 (d.h. für den jeweiligen Zufuhr- und Abflussanschluss) besonders geeignet. Mit anderen Worten kann die Vorrichtung 1 mit einem Zufuhranschluss und einem Abflussanschluss für die zweiten Kammern 7 an gegenüberliegenden Seiten des quadratischen Gehäuses 2 versehen sein, aber auch mit einem Zufuhranschluss und einem Abflussanschluss für die dritten Kammern 13 an den anderen gegenüberliegenden Seiten des Gehäuses 2. Zudem kann das Gehäuse 2 noch einen Querstrom der biologischen Flüssigkeit durch die jeweiligen Membranlagen bzw. -matten 19, 21 zulassen, wie in Figur 3 schematisch gezeigt ist.

Zweck der vorhergehenden Beschreibung ist die Wirkungsweise bevorzugter Ausführungen der Erfindung zu illustrieren, und nicht der Umfang der Erfindung einzuschränken. Ausgehend von der vorhergehenden Erläuterung werden einem Fachmann viele Variationen deutlich sein, die vom Offenbarungsgehalt der vorliegenden Erfindung erfasst. Wie der Fachmann zum Beispiel verstehen wird, müssen die oben beschriebenen Hohlfasermatten 19, 21 nicht immer abwechselnd gestapelt werden. Die jeweiligen Mattenarten 19, 21 können z.B. in gleichartigen Gruppen gestapelt und kombiniert werden, und diese Gruppen dann später miteinander kombiniert werden. Wie der Fachmann verstehen wird, ist die Vorrichtung 1 nicht auf maximale zwei Behandlungen der biologischen Flüssigkeit begrenzt. Vielmehr lässt der Aufbau der Vorrichtung 1 eine Mehrzahl von möglichen gleichzeitigen Behandlungen zu.

### Experimentelle Ergebnisse bei Tierversuchen

In einem Pilotexperiment an vier Versuchstieren (Schweine) wurde eine erfindungsgemäße Vorrichtung nach dem in Figur 3 beschriebenen Ausführungsbeispiel zur Behandlung des Blutes, durch einen Gasaustausch (O₂ und CO₂) und Hämofiltraton, an jedes Tier angeschlossen und über einen Zeitraum von 6 Stunden betrieben. Die erfindungsgemäßen Vorrichtungen waren in der Form von Test-Modulen oder Prototypen und die Beatmung, Blutdruck und Durchfluss durch den Test-Modul für jedes Tier wurde permanent kontrolliert. In Abständen von 30 Minuten wurden Blutproben zur Serum- und Blutgasanalyse entnommen. Die Tiere konnten über einen Zeitraum von 6 Stunden mit den Test-Modulen (also, den Prototypen) beatmet werden.

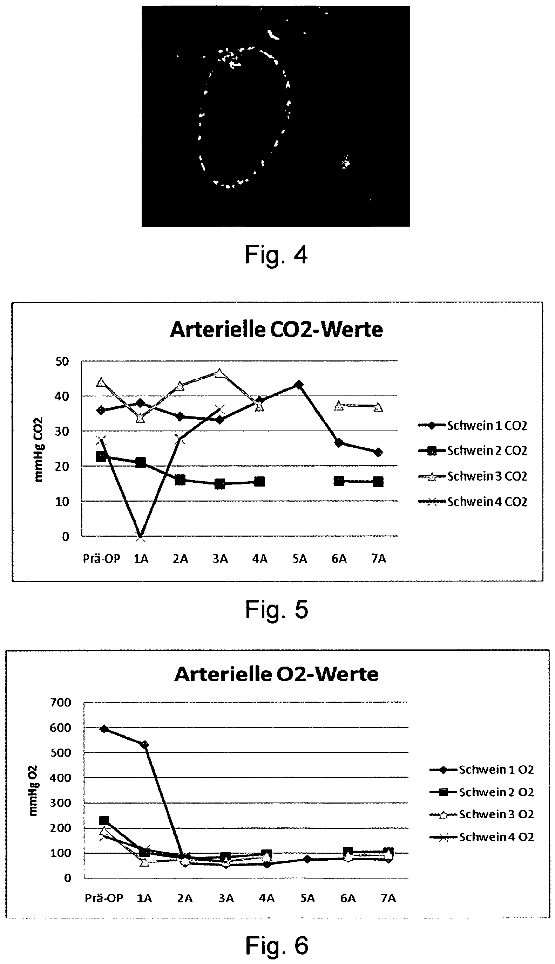

Zur Durchführung der Tierexperimente waren zwei von den vier Modulen (nämlich für die Tiere 2 und 4) mit Zellen besiedelt worden. Hierfür wurden in einem Zellkulturlabor A549 Zellen in Kultur genommen und über mehrere Wochen expandiert. Nach erreichen der nötigen Zellzahl wurden die Zellen durch Trypsinbehandlung abgelöst, in 40ml Medium aufgenommen und in die PES-Fasern der Prototypen injeziert. Nach einer Inkubation von 2-3 Tagen waren die Zellen im Inneren der Fasern adhäriert. Dies konnte durch Kernfärbung mit dem Farbstoff DAPI an Kryotomschnitten von Fasern nachgewiesen werden, wie in Figur 4 gezeigt. Der Blutfluss durch besiedelte und unbesiedelte Module war mit 1,1 vs. 1,3 Litern Blut je Minute vergleichbar, die Besiedlung bzw. Vorinkubation der Prototypen hatte keinen wesentlichen Einfluss auf die Blutflussraten.

Wie den Figuren 5 und 6 zu entnehmen ist, zeigte sich bei den Arteriellen Blutgaswerten bei CO2 (Figur 5) und O₂ (Figur 6) ein recht gleichbleibendes Niveau über den Versuchszeitraum hinweg. Bei Schwein 2 sank der CO₂-Wert innerhalb der ersten drei Messungen von ca. 22 mmHg auf ca. 15 mmHg ab. Die arteriellen O₂-Werte lagen nach Anschluss der Prototypen in einem Bereich von 50-100 mmHg.

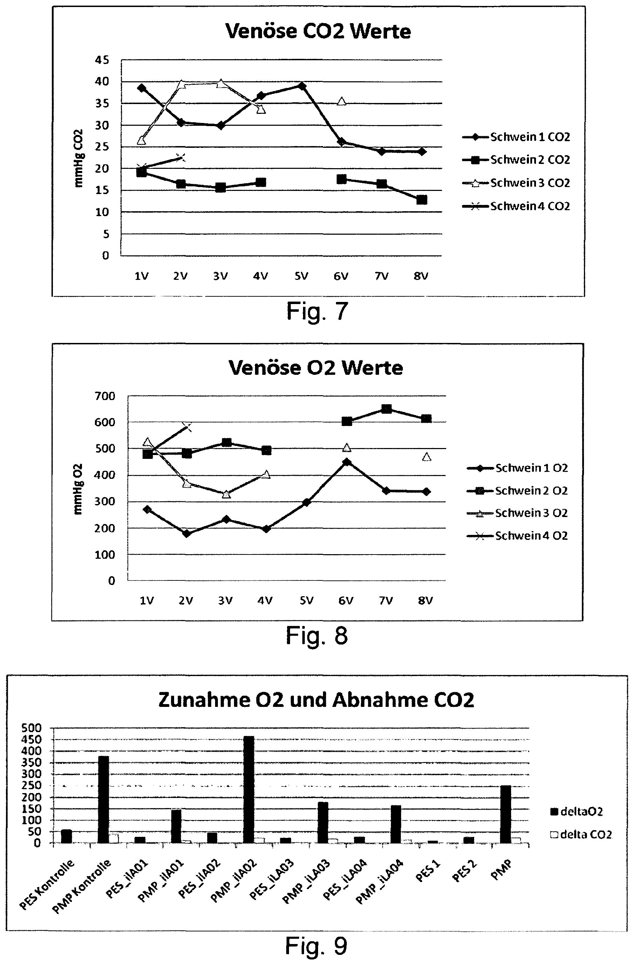

Aus den Figuren 7 und 8 geben die Partialdrücke im venösen Blut Aufschluss über die Gastransferleistung in den Prototypen. Hier fällt auf, dass die venösen CO2 Werte (Figur 7) nur geringfügig unter den arteriellen Werten liegen. Die allgemein niedrigen Werte lassen auf eine relativ hohe und konstante CO₂-Elimination schließen. Die Venösen O₂-Werten (Figur 8) weisen eine größere Spannweite auf. Tier 1 hatte die niedrigesten O₂-Partialdrücke (200-330 mmHg) während die Schweine 2 und 4 relativ hohe O₂ Partialdrücke von über 500 mmHg aufwiesen. Dies zeigt die höhere Gastransferleistung der besiedelten Test-Module, wobei sowohl die Zellularisierung als auch die Vorinkubation in serumhaltigem Medium Einfluss auf die Verbesserte Gastransferleistung haben können. In Figur 9 ist die deutliche Zunahme von O₂ im Blut über die (zweiten) PMP-Membranen jedes Moduls (als 01 bis 04 gekennzeichnet) gezeigt.

Aufgrund der Gefäßdrücke konnte beobachtet werden, dass Serum durch die PES-Membranen in die dritten Kammern 13, also in die Lumenseite der Hohlfasern 14 gelangte. Hiermit konnte gezeigt werden, dass die Fasern durchlässig für Plasma sind und Zellen auf der Lumenseite versorgt werden können. Bei Schwein 3 zeigte sich ein etwas höherer Druckabfall über den Modul als bei Schwein 1, allerdings lagen beide Werte auf einem sehr niedrigen Niveau. Daher lagen die Druckabfälle über die Module in einem akzeptablen bereich.

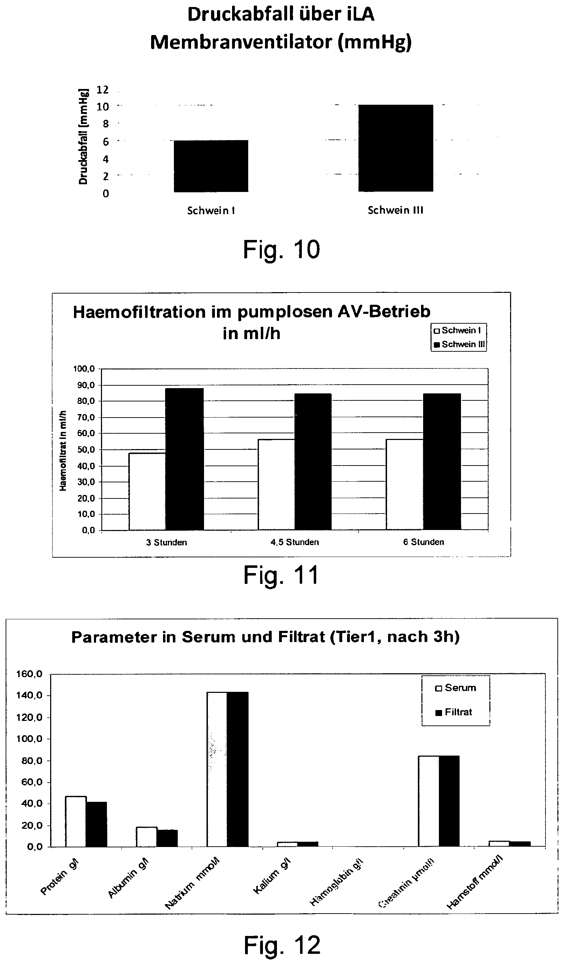

Mit Bezug auf die Figur 10 zeigte die Haemofiltrationsleistung der PES-Faser (durch die Membranen 15 in die dritten Kammern 13) über den gesamten Zeitraum von 6 Stunden gleichbleibende Werte. Mit Bezug auf die Figur 11 fiel es jedoch auf, dass interindividuelle Unterschiede (hier zwischen den Modulen für Tier 1 und Tier 3) relativ hoch ausfielen. Dies ist zum einen auf den unterschiedlichen Gefäßdruck zurückzuführen, als auch auf die hämostatische Situation und den Flüssigkeiststatus der Tiere.

Wie der Figur 12 zu entnehmen ist, unterschieden sich die Filtratparameter nur unwesentlich von den Serumparametern für Tier 1. Somit kann von einer sehr guten Haemofiltrationsleistung ausgegangen werden.

## Patentansprüche

1. Vorrichtung (1) zur Behandlung einer biologischen Flüssigkeit, umfassend eine erste Kammer (3), die zur Aufnahme der biologischen Flüssigkeit bestimmt ist, und eine zweite Kammer (7), die zur Aufnahme eines Gases bestimmt ist, wobei die erste Kammer (3) und die zweite Kammer (7) durch mindestens eine gasdurchlässige und flüssigkeitsundurchlässige Membran (9) voneinander getrennt sind, durch welche Membran (9) Gasmoleküle zwischen der ersten Kammer (3) und der zweiten Kammer (7) zur Behandlung der biologischen Flüssigkeit transferierbar sind und wobei die zweite Kammer (7) in mehreren Kammern unterteilt ist, sodass die Vorrichtung (1) mehrere zweite Kammern (7) umfasst, die zur Aufnahme eines Gases bestimmt und von der ersten Kammer (3) durch eine gasdurchlässige und flüssigkeitsundurchlässige Membran (9) getrennt sind, wobei die mehreren zweiten Kammern (7) sich innerhalb der ersten Kammer befinden bzw. von der ersten Kammer (3) im Wesentlichen umgeben sind, wobei die Vorrichtung ferner eine dritte Kammer (3) umfasst, die durch mindestens eine flüssigkeitsdurchlässige Membran (15) von der ersten Kammer (3) getrennt ist und einem Entzug einer oder mehrerer Komponenten der biologischen Flüssigkeit dient, wobei die dritte Kammer (13) in mehreren Kammern unterteilt ist, sodass die Vorrichtung (1) mehrere dritte Kammern (13) umfasst, die durch eine flüssigkeitsdurchlässige Membran (15) von der ersten Kammer (3) getrennt sind und zum Entzug einer oder mehrerer Komponenten der biologischen Flüssigkeit dienen, wobei die mehreren dritten Kammern (13) sich innerhalb der ersten Kammer befinden bzw. von der ersten Kammer (3) im Wesentlichen umgeben sind.

2. Vorrichtung (1) nach Anspruch 1, **dadurch gekennzeichnet, dass** die zweite Kammer (7) von der mindestens einen gasdurchlässigen und flüssigkeitsundurchlässigen Membran (9) abgegrenzt bzw. umschlossen ist.

3. Vorrichtung (1) nach Anspruch 1 oder 2, **dadurch gekennzeichnet, dass** die dritte Kammer (13) von der mindestens einen flüssigkeitsdurchlässigen Membran (15) abgegrenzt bzw. umschlossen ist.

4. Vorrichtung (1) nach einem der vorhergehenden Ansprüche, **dadurch gekennzeichnet, dass** die dritte Kammer (13):
für einen Unterdruck bezüglich der ersten Kammer (3) bestimmt ist, um einen Transfer der Komponente(n) aus der biologischen Flüssigkeit in die dritte Kammer (13) zu befördern, und/oder
zur Aufnahme einer Flüssigkeit bestimmt ist, um einen Transfer der Komponente(n) aus der biologischen Flüssigkeit in die dritte Kammer (13) zu befördern.

5. Vorrichtung (1) nach einem der vorhergehenden Ansprüche, **dadurch gekennzeichnet, dass** die zweite(n) Kammer(n) (7) einen länglichen und bevorzugt im Wesentlichen zylinderförmigen Aufbau aufweist bzw. aufweisen, der im Querschnitt einen oder mehrere durchgängige Hohlräume aufweist, und eine den Querschnitt abgrenzende Wand (8) der zweiten Kammer(n) (7) die gasdurchlässige und flüssigkeitsundurchlässige Membran (9) mindestens teilweise bildet,
wobei vorzugsweise die mehreren zweiten Kammern (7) in einer oder mehreren Reihen nebeneinander und bevorzugt in einem Abstand (D₂) voneinander angeordnet sind, und die mehreren zweiten Kammern (7) bevorzugt in mehreren Lagen (19) angeordnet sind.

6. Vorrichtung (1) nach einem der vorhergehenden Ansprüche, **dadurch gekennzeichnet, dass** die dritte(n) Kammer(n) (13) einen länglichen und bevorzugt im Wesentlichen zylinderförmigen Aufbau aufweist bzw. aufweisen, der im Querschnitt einen oder mehrere durchgängige Hohlräume aufweist, und dass eine den Querschnitt abgrenzende Wand (14) der dritten Kammer(n) (13) die flüssigkeitsdurchlässige Membran (15) mindestens teilweise bildet.

7. Vorrichtung (1) nach einem der vorhergehenden Ansprüche, **dadurch gekennzeichnet, dass** die mehreren dritten Kammern (13) in einer oder mehreren Reihen nebeneinander und bevorzugt in einem Abstand (D₃) voneinander angeordnet sind, und dass die mehreren dritten Kammern (13) bevorzugt in mehreren Lagen (21) angeordnet sind.

8. Vorrichtung (1) nach einem der vorhergehenden Ansprüche, **dadurch gekennzeichnet, dass**:
die zweite(n) Kammer(n) (7) und/oder die dritte(n) Kammer(n) (13) als Hohlkörper, bevorzugt als Hohlfaser(n), ausgebildet ist bzw. sind, sodass eine Wand (8, 14) des Hohlkörpers bzw. der Hohlfaser die jeweilige Membran (9, 15) bildet, und/oder
eine längliche Ausrichtung der zweiten Kammer(n) (7) sich quer und bevorzugt rechtwinkelig zu einer länglichen Ausrichtung der dritten Kammer(n) (13) erstreckt.

9. Vorrichtung (1) nach einem der vorhergehenden Ansprüche, **dadurch gekennzeichnet, dass** die erste Kammer (3) als Durchflusskammer gestaltet ist und für einen Durchfluss von einem Einlass (5) zu einem Auslass (6) in einer Richtung (4) gegen oder quer zu der zweiten bzw. dritten Kammer(n) (7, 13) ausgebildet ist.

10. Vorrichtung (1) nach einem der vorhergehenden Ansprüche, **dadurch gekennzeichnet, dass** mindestens eine der Membranen (9, 15) aus einem organischen Material besteht oder ein solches umfasst, wobei das organische Material bevorzugt ein Polymer, Polymerkomposit oder Polymerschichtung ist, wobei:
das Material der mindestens einen gasdurchlässigen und flüssigkeitsundurchlässigen Membran (9) vorzugsweise ein Polyolefin ist, wobei das Polyolefin bevorzugt Polymethylpenten ist, und/oder
das Material der mindestens einen flüssigkeitsdurchlässigen Membran (15) vorzugsweise Polyethersulfone ist.

11. Vorrichtung (1) nach einem der vorhergehenden Ansprüche, **dadurch gekennzeichnet, dass** die Membranen (9, 15) eine Stärke von 10 bis 100 µm, vorzugsweise von 20 bis 50 µm aufweisen.

12. Vorrichtung (1) nach einem der vorhergehenden Ansprüche, **dadurch gekennzeichnet, dass** jede zweite bzw. dritte Kammer (7, 13) einen Durchmesser im Bereich von 1 µm bis 1 cm aufweist.

13. Vorrichtung (1) nach einem der vorhergehenden Ansprüche, **dadurch gekennzeichnet, dass** mindestens eine der Membranen (9, 15):
und vorzugsweise jede Membran, durch ein Trägermaterial stabilisiert ist und/oder
bevorzugt die mindestens eine gasdurchlässige und flüssigkeitsundurchlässige Membran (9), mit Zellen, vorzugsweise Epithelzellen, besiedelt ist und/oder mit einer oder mehreren Substanzen, ausgewählt aus der Gruppe, bestehend aus (Poly)Saccharid, vorzugsweise Heparin, Nukleinsäure, Protein, vorzugsweise Albumin, beschichtet ist.

14. Vorrichtung (1) nach einem der vorhergehenden Ansprüche, **dadurch gekennzeichnet, dass** die mindestens eine gasdurchlässige und flüssigkeitsundurchlässige Membran (9) selektiv im Wesentlichen für Sauerstoff und/oder Kohlenstoffdioxid durchlässig ist und/oder nicht oder nur geringfügig für Stickstoff durchlässig ist.

15. Vorrichtung (1) nach einem der vorhergehenden Ansprüche, **dadurch gekennzeichnet, dass** die mindestens eine flüssigkeitsdurchlässige Membran für ein Hämodialyse- und/oder ein Hämofiltrationsverfahren geeignet ist.

16. Vorrichtung (1) nach einem der vorhergehenden Ansprüche, **dadurch gekennzeichnet, dass** die biologische Flüssigkeit ausgewählt ist aus der Gruppe, bestehend aus Blut, Blutserum, Zellsuspension, Zelllösung und Kulturmedium.

17. Vorrichtung, bestehend aus zwei oder mehreren der Vorrichtungen (1) nach einem der vorhergehenden Ansprüche.

## Claims

1. Device (1) for the treatment of a biological fluid, comprising a first chamber (3) which is adapted for receiving the biological fluid, and a second chamber (7) which is adapted for receiving a gas, wherein the first chamber (3) and the second chamber (7) are separated from each other by at least one gas-permeable and liquid-impermeable membrane (9), through which membrane (9) gas molecules are transferable between the first chamber (3) and the second chamber (7) for the treatment of the biological fluid, and wherein the second chamber (7) is divided into several chambers, such that the device (1) comprises a plurality of second chambers (7) which are adapted for receiving a gas, and which are separated from the first chamber (3) by a gas-permeable and liquid-impermeable membrane (9), wherein the plurality of second chambers (7) are located within the first chamber or are substantially surrounded by the first chamber (3), and wherein the device further comprises a third chamber (13) which is separated from the first chamber (3) by at least one liquid-permeable membrane (15), and which is adapted for a withdrawal of one or more components of the biological fluid, wherein the third chamber (13) is divided into several chambers, such that the device (1) comprises a plurality of third chambers (13), which are separated from the first chamber (3) by a liquid-permeable membrane (15) and are adapted for the withdrawal of one or more components of the biological fluid, wherein the plurality of third chambers (13) are located within the first chamber or are substantially surrounded by the first chamber (3).

2. Device (1) according to claim 1, **characterized in that** the second chamber (7) is separated from or surrounded by the at least one gas-permeable and liquid-impermeable membrane (9).

3. Device (1) according to claim 1 or 2, **characterized in that** the third chamber (13) is separated from or surrounded by the at least one liquid-permeable membrane (15).

4. Device (1) according to one of the preceding claims, **characterized in that** the third chamber (13) is adapted:
for a negative pressure relative to the first chamber (3) to promote a transfer of the component(s) from the biological fluid into the third chamber (13), and/or is adapted for receiving a liquid to promote a transfer of the component(s) from the biological fluid into the third chamber (13).

5. Device (1) according to one of the preceding claims, **characterized in that** the second chamber(s) (7) has/have an elongated and preferably substantially cylindrical structure, which comprises in a cross-section one or more continuous cavities, and that a wall (8) of the second chamber(s) (7) demarcating the cross-section at least partially forms the gas-permeable and liquid-impermeable membrane (9),
wherein the plurality of second chambers (7) are preferably arranged in one or more rows side by side and preferably in a distance (D₂) from each other, and that the plurality of second chambers (7) are preferably arranged in several layers (19).

6. Device (1) according to one of the preceding claims, **characterized in that** the third chamber(s) (13) has/have an elongated and preferably substantially cylindrical structure which comprises in a cross-section one or more continuous cavities, and that a wall (14) of the third chamber(s) (13) demarcating the cross-section at least partially forms the liquid-permeable membrane (15).

7. Device (1) according to one of the preceding claims, **characterized in that** the plurality of third chambers (13) are arranged in one or more rows side by side and preferably in a distance (D₃) from each other, and that the plurality of third chambers (13) are preferably arranged in several layers (21).

8. Device (1) according to one of the preceding claims, **characterized in that**:
the second chamber(s) (7) and/or the third chamber(s) (13) is/are formed as hollow body/bodies, preferably hollow fibre(s), such that a wall (8, 14) of the hollow body or hollow fibre forms the respective membrane (9, 15), and/or
an elongated alignment of the second chamber(s) (7) extends transversely and preferably in a right angle to an elongated alignment of the third chamber(s) (13).

9. Device (1) according to one of the preceding claims, **characterized in that** that the first chamber (3) is formed as a flow chamber and designed for a flow from an inlet (5) to an outlet (6) in a direction (4) opposite or transversely to the second or third chamber(s) (7, 13).

10. Device (1) according to one of the preceding claims, **characterized in that** at least one of the membranes (9, 15) is made of or comprises an organic material, wherein the organic material preferably is a polymer, a polymer composite or polymer layer, wherein
the material of the at least one gas-permeable and liquid-impermeable membrane (9) is preferably a polyolefin, wherein the polyolefin preferably is polymethylpentene, and/or the material of the at least one liquid-permeable membrane (15) is preferably polyethersulfone.

11. Device (1) according to one of the preceding claims, **characterized in that** the membranes (9, 15) have a thickness of 10 to 100 µm, preferably of 20 to 50 µm.

12. Device (1) according to one of the preceding claims, **characterized in that** each of the second and/or third chambers (7, 13) has a diameter ranging from 1 µm to 1 cm.

13. Device (1) according to one of the preceding claims, **characterized in that** the at least one of the membranes (9, 15):
and preferably all of the membranes, is/are stabilized by a support material, and/or
preferably the at least one of the gas-permeable and liquid-impermeable membrane (9) is populated with cells, preferably epithelial cells, and/or
is coated with one or more substances selected from the group consisting of (poly)saccharide, preferably heparin, nucleic acid, protein, preferably albumin.

14. Device (1) according to one of the preceding claims, **characterized in that** the at least one gas-permeable and fluid-impermeable membrane (9) is selectively permeable in particular for oxygen and/or carbon dioxide, and/or is impermeable or only slightly permeable for nitrogen.

15. Device (1) according to one of the preceding claims, **characterized in that** the at least one fluid-permeable membrane is suitable for a hemodialysis and/or hemofiltration procedure.

16. Device (1) according to one of the preceding claims, **characterized in that** the biological fluid is selected from the group consisting of blood, blood serum, cell suspension, cell solution and culture medium.

17. Device consisting of two or more devices (1) according to one of the preceding claims.

## Revendications

1. Dispositif (1) pour le traitement d'un liquide biologique, comprenant une première chambre (3), qui est destinée à recevoir le liquide biologique, et une deuxième chambre (7), qui est destinée à recevoir un gaz, la première chambre (3) et la deuxième chambre (7) étant séparées l'une de l'autre par au moins une membrane (9) perméable aux gaz et imperméable aux liquides, via laquelle membrane (9) des molécules de gaz sont transférables entre la première chambre (3) et la deuxième chambre (7) pour le traitement du liquide biologique et la deuxième chambre (7) étant sous-divisée en plusieurs chambres de telle sorte que le dispositif (1) comprend plusieurs deuxièmes chambres (7), qui sont destinées à recevoir un gaz et qui sont séparées de la première chambre (3) par une membrane (9) perméable aux gaz et imperméable aux liquides, lesdites plusieurs deuxièmes chambres (7) se trouvant dans la première chambre ou étant pratiquement entourées par la première chambre (3), le dispositif comprenant en outre une troisième chambre (13), qui est séparée de la première chambre (3) par au moins une membrane (15) perméable aux liquides et qui sert au retrait d'un ou de plusieurs composants du liquide biologique, la troisième chambre (13) étant sous-divisée en plusieurs chambres de telle sorte que le dispositif (1) comprend plusieurs troisièmes chambres (13) qui sont séparées de la première chambre (3) par une membrane (15) perméable aux liquides et qui servent au retrait d'un ou de plusieurs composants du liquide biologique, lesdites plusieurs troisièmes chambres (13) se trouvant dans la première chambre ou étant pratiquement entourées par la première chambre (3).

2. Dispositif (1) selon la revendication 1, **caractérisé en ce que** la deuxième chambre (7) est délimitée ou entourée par ladite au moins une membrane (9) perméable aux gaz et imperméable aux liquides.

3. Dispositif (1) selon la revendication 1 ou 2, **caractérisé en ce que** la troisième chambre (13) est délimitée ou entourée par ladite au moins une membrane (15) perméable aux liquides.

4. Dispositif (1) selon l'une quelconque des revendications précédentes, **caractérisé en ce que** la troisième chambre (13) :
- est destinée à une dépression par rapport à la première chambre (3) pour favoriser un transfert du/des composant(s) à partir du liquide biologique dans la troisième chambre (13) et/ou
- est destinée à recevoir un liquide pour favoriser un transfert du/des composant(s) à partir du liquide biologique dans la troisième chambre (13).

5. Dispositif (1) selon l'une quelconque des revendications précédentes, **caractérisé en ce que** la/les deuxième(s) chambre(s) (7) présent(ent) une structure allongée et de préférence sensiblement cylindrique, qui présente un ou plusieurs espaces creux traversants en section transversale et une paroi (8) délimitant la section transversale de la/des deuxième(s) chambre(s) (7) formant au moins partiellement la membrane (9) perméable aux gaz et imperméable aux liquides, lesdites plusieurs deuxièmes chambres (7) étant de préférence disposées en une ou plusieurs rangées les unes à côté des autres et de préférence disposées à une certaine distance (D₂) les unes des autres et lesdites plusieurs deuxièmes chambres (7) étant de préférence disposées en plusieurs couches (19).

6. Dispositif (1) selon l'une quelconque des revendications précédentes, **caractérisé en ce que** la/les troisième(s) chambre(s) (13) présent(ent) une structure allongée et de préférence sensiblement cylindrique, qui présente un ou plusieurs espaces creux traversants en section transversale et **en ce qu'**une paroi (14) délimitant la section transversale de la/des troisième(s) chambre(s) (13) forme au moins partiellement la membrane (15) perméable aux liquides.

7. Dispositif (1) selon l'une quelconque des revendications précédentes, **caractérisé en ce que** lesdites plusieurs troisièmes chambres (13) sont disposées en une ou plusieurs rangées les unes à côté des autres et de préférence disposées à une certaine distance (D₃) les unes des autres et **en ce que** lesdites plusieurs troisièmes chambres (13) sont de préférence disposées en plusieurs couches (21).

8. Dispositif (1) selon l'une quelconque des revendications précédentes, **caractérisé en ce que** :
- la/les deuxième(s) chambre(s) (7) et/ou la/les troisième(s) chambre(s) (13) est/sont conçue(s) sous forme de corps creux, de préférence sous forme de fibre(s) creuse(s) de telle sorte qu'une paroi (8, 14) du corps creux ou de la fibre creuse forme la membrane (9, 15) respective et/ou
- une orientation longitudinale de la/des deuxième(s) chambre(s) (7) s'étend transversalement et de préférence perpendiculairement à une orientation longitudinale de la/des troisième(s) chambre(s) (13).

9. Dispositif (1) selon l'une quelconque des revendications précédentes, **caractérisé en ce que** la première chambre (3) est réalisée comme chambre à écoulement et est conçue pour un écoulement à partir d'une entrée (5) vers une sortie (6) dans une direction (4) opposée ou transversale par rapport à la/aux deuxième(s) ou troisième(s) chambres (7, 13).

10. Dispositif (1) selon l'une quelconque des revendications précédentes, **caractérisé en ce qu'**au moins l'une des membranes (9, 15) est constituée par un matériau organique ou comprend un tel matériau, le matériau organique étant de préférence un polymère, un composite polymère ou un stratifié polymère :
- le matériau de ladite au moins une membrane (9) perméable aux gaz et imperméable aux liquides étant de préférence une polyoléfine, la polyoléfine étant de préférence le polyméthylpentène et/ou
- le matériau de ladite au moins une membrane perméable aux liquides (15) étant de préférence une polyéthersulfone.

11. Dispositif (1) selon l'une quelconque des revendications précédentes, **caractérisé en ce que** les membranes (9, 15) présentent une épaisseur de 10 à 100 µm, de préférence de 20 à 50 µm.

12. Dispositif (1) selon l'une quelconque des revendications précédentes, **caractérisé en ce que** chaque deuxième ou troisième chambre (7, 13) présente un diamètre dans la plage de 1 µm à 1 cm.

13. Dispositif (1) selon l'une quelconque des revendications précédentes, **caractérisé en ce qu'**au moins l'une des membranes (9, 15) :
et de préférence chaque membrane, est stabilisée par un matériau support et/ou
de préférence, ladite au moins une membrane (9) perméable aux gaz et imperméable aux liquides est ensemencée par des cellules de préférence des cellules épithéliales et/ou
est revêtue par une ou plusieurs substances, choisies dans le groupe constitué par un (poly)saccharide, de préférence l'héparine, un acide nucléique, une protéine, de préférence l'albumine.

14. Dispositif (1) selon l'une quelconque des revendications précédentes, **caractérisé en ce que** ladite au moins une membrane (9) perméable aux gaz et imperméable aux liquides est sélectivement perméable essentiellement à l'oxygène et/ou au dioxyde de carbone et/ou n'est pas, ou seulement peu, perméable à l'azote.

15. Dispositif (1) selon l'une quelconque des revendications précédentes, **caractérisé en ce que** ladite au moins une membrane perméable aux liquides est appropriée pour un procédé d'hémodialyse et/ou d'hémofiltration.

16. Dispositif (1) selon l'une quelconque des revendications précédentes, **caractérisé en ce que** le liquide biologique est choisi dans le groupe constitué par le sang, le sérum sanguin, une suspension cellulaire, une solution cellulaire et un milieu de culture.

17. Dispositif constitué par deux des dispositifs (1) ou plus selon l'une quelconque des revendications précédentes.
